# EUROPEAN PATENT SPECIFICATION

(11) **EP 1 868 639 B1**
(45) Date of publication and mention of the grant of the patent: **25.08.2010**
(21) Application number: 06748299.2
(22) Date of filing: 06.03.2006
(51) Int. Cl.: A61K 39/00, A61K 39/42, A61K 39/395

(54) **METHODS OF APPLYING IONIZATION RADIATION FOR THERAPY OF HIV INFECTION**
VERFAHREN ZUR ANWENDUNG VON IONISIERUNGSSTRAHLUNG BEI EINER HIV-INFEKTIONSTHERAPIE
PROCEDES D'APPLICATION DE RAYONS D'IONISATION POUR LA THERAPIE DE L'INFECTION VIH

(30) Priority: 07.03.2005 US 659582 P
(43) Date of publication of application: 26.12.2007
(73) Proprietor: ALBERT EINSTEIN COLLEGE OF MEDICINE OF YESHIVA UNIVERSITY, Bronx, New York 10461 (US)
(72) Inventor: DADACHOVA, Ekaterina, Mahopac, New York 10541 (US); CASADEVALL, Arturo, New Rochelle, New York 10801-6392 (US); GOLDSTEIN, Harris, Teaneck, New Jersey 07666 (US)
(74) Representative: Paemen, Liesbet R.J.
(86) International application number: PCT/US2006/007961
(87) International publication number: WO 2006/096656

(56) References cited:
- US-A1- 2004 115 203
- HERNIGOU PHILIPPE ET AL: "Inactivation of HIV by application of heat and radiation: Implication in bone banking with irradiated allograft bone" ACTA ORTHOPAEDICA SCANDINAVICA, vol. 71, no. 5, October 2000 (2000-10), pages 508-512, XP002521998 ISSN: 0001-6470
- GRIFFITHS GARY L ET AL: "Technetium-99m, rhenium-186, and rhenium-188 direct-labeled antibodies" CANCER (PHILADELPHIA), vol. 73, no. 3 SUPPL., 1994, pages 761-768, XP002521999 ISSN: 0008-543X
- CASADEVALL ET AL.: 'Passive antibodytherapy for infectious diseases' NATURE REVIEWS MICROBIOLOGY vol. 2, September 2004, pages 695 - 703, XP008080877
- DADACHOVA ET AL.: 'Targeted killing of virally infected cells by radiolabeled antibodies to viral proteins' PLOS MED. vol. 3, no. 11, November 2006, pages 1 - 10
- DADACHOVA ET AL.: 'Feasibility of radioimmunotherapy of experimental pneumonoclonal infection' ANTIMICROBIAL AGENTS AND CHEMOTHERAPY vol. 48, no. 5, May 2004, pages 1624 - 1629, XP008090893
- WALDMANN: 'Immunotherapy: past, present and future' NATURE MEDICINE vol. 9, no. 3, March 2003, pages 269 - 277, XP003020016

## Description

### CROSS-REFERENCE TO RELATED APPLICATION

This application claims the benefit of U.S. Provisional Application No. 60/659,582, filed March 7, 2005, the content of which is hereby incorporated by reference in its entirety into the subject application.

### STATEMENT OF GOVERNMENT SUPPORT

The invention disclosed herein was made with U.S. Government (National Institutes of Health) support under Albert Einstein College of Medicine (AECOM) Center for AIDS Research grant number 9526-9814 and by grant numbers AI60507, AI033142, AI033774 and HL059842. Accordingly, the U.S. Government has certain rights in this invention.

### FIELD OF THE INVENTION

The present invention relates to the treatment of HIV infection using radioimmunotherapy.

### BACKGROUND OF THE INVENTION

Throughout this application various publications are referred to in parenthesis. Full citations for these references may be found at the end of the specification immediately preceding the claims. The disclosures of these publications are hereby incorporated by reference in their entireties into the subject application to more fully describe the art to which the subject application pertains.

The human immunodeficiency virus (HIV) epidemic is a major threat to health in the developing and western world. HIV induces acquired immune deficiency syndrome (AIDS). An estimated 40 million people world-wide are infected with the virus. After more than 20 years into the epidemic, not a single person has been cured of the infection (Hamer, 2004). Highly active antiretroviral therapy (HAART), a combination of drugs that inhibits enzymes essential for HIV replication, can reduce the viremia and opportunistic infections in most patients, and prolong survival. However, HAART regimens are expensive, complicated and often accompanied by significant toxicity (Carr, 2003). Furthermore, the virus persists in infected cells (Chun et al., 1997), and HIV can rapidly evolve resistance to HAART drugs (Little et al., 2002). It has been argued that latent HIV infection in cellular reservoirs renders the infection intrinsically incurable by antiretroviral therapy alone (Persaud et al., 2003). Hence, HIV infection is often manageable but not curable. To combat this problem, several approaches have been tried (Hamer, 2004), among them the use of immunotoxins that specifically recognize HIV-encoded membrane proteins and thereby potentiate the destruction of infected cells (Bera et al., 1998; Goldstein et al., 2000; Pincus et al., 2003; Saavedra-Lozano et al., 2002, 2004; Till et al., 1987). Although promising, none of these strategies has yet been shown to be effective in humans, and there is theoretical concern for the suitability of those approaches for repeated dosing. Clinical trials of the toxin CD4 - *Pseudomonas* exotoxin (CD4-PE), which targets the HIV envelope glycoprotein gp120, were not successful due to high nonspecific toxicity and lack of therapeutic effect at maximum tolerated doses (Davey et al., 1994; Ramachandran et al., 1994). Thus, there remains a long-felt need for treatment of individuals with HIV infection, especially for new treatment options.

Radioimmunotherapy (RIT) is a therapeutic modality which uses antibody-antigen interaction and antibodies radiolabeled with therapeutic radioisotopes. Radiolabeled antibodies have been used to treat experimental murine cryptococcosis and pneumococcal bacterial infection (Dadachova et al., 2003, 2004a-c; U.S. Patent Application Publication No. US 2004/0115203). However, since HIV (Hernigou et al., 2000), and certain other types of microorganisms (e.g., many fungi, bacterium *Deinococcus radiodurans,* and yeasts *Saccharomyces ellipsoideus* and *Saccharomyces cerevisiae* (Casarett, 1968; Komarova et al., 2002; Mironenko et al., 2000; Sayeg et al., 1959; Schmidt et al., 2002; Shvedenko et al., 2001)), are extremely resistant to gamma radiation, it has not been apparent whether or not HIV would be susceptible to radioimmunotherapy. In addition, antibody-dependent enhancement of HIV infection has been reported (Robinson et al., 1990, 1991). Furthermore, antibodies to *C*. *neoformans* radiolabeled with 125-Iodine are known to quickly lose their radiolabel *in vivo* (Goldman et al., 1997). Accordingly, the likelihood of success of using radioimmunotherapy to treat individuals infected with HIV was not apparent prior to the present disclosure.

### SUMMARY OF THE INVENTION

The present invention is directed to the combination of immune and radiation therapy for the treatment of human immunodeficiency virus (HIV) infection. Surprisingly, it was found that although radiolabeled antibodies to HIV envelope proteins are not effective at killing HIV particles, such therapy is effective at killing cells that harbor HIV. The present invention, which targets and kills HIV infected cells, is expected to have a major impact on the treatment of acute HIV exposure and elimination of persistent reservoirs of HIV-infected cells, which serve as sites of viral synthesis and latency.

The present invention provides a method for treating a subject infected with HIV which comprises administering to the subject an amount of a radiolabeled antibody effective to kill HIV infected cells, wherein the antibody is specific for a HIV envelope glycoprotein and wherein the radiolabeled antibody specifically binds to cells that are infected with HIV virus and that express the HIV envelope glycoprotein to which the antibody specifically binds.

The invention also provides a pharmaceutical composition formulated in dosage form, comprising a radiolabeled antibody and a pharmaceutically acceptable carrier, wherein the antibody is specific for a HIV envelope glycoprotein and the dosage is appropriate to kill cells infected with HIV in a subject.

### BRIEF DESCRIPTION OF THE FIGURES

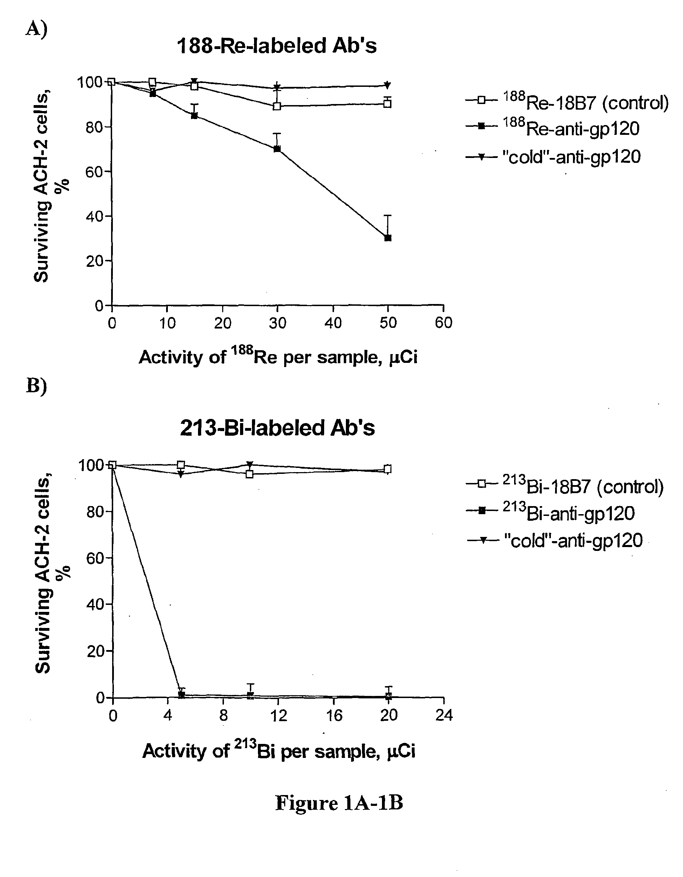

Figure 1A-1B. *In vitro* killing of ACH-2 cells with ¹⁸⁸Re-and ²¹³Bi-labeled anti-gp120 antibody. A) ACH-2 cells treated with anti-gp120 ¹⁸⁸Re-Ab; B) ACH-2 cells treated with anti-gp120 ²¹³Bi-Ab. In A-B matching amounts (2.5-12.5 µg) of "cold" Abs were used.

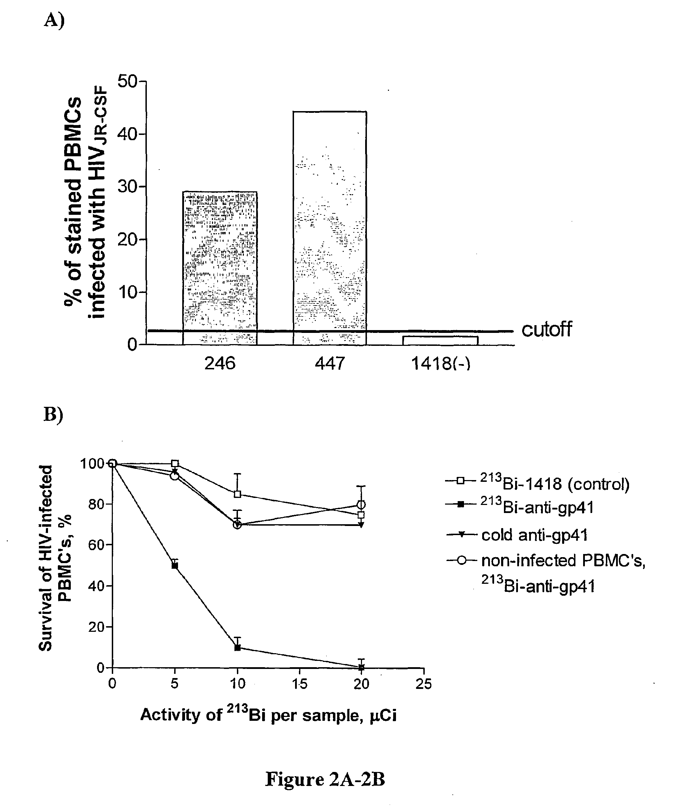

Figure 2A-2B. *In vitro* binding and killing of human peripheral blood mononuclear cells (PBMCs) with ²¹³Bi-labeled radiolabeled Ab. A) Binding of human mAbs to the surface of PBMCs infected with the JR-CSF strain of HIV-1 by flow cytometry. Human mAb 1418 (IgG1) to parvovirus B19 (Gigler et al., 1999) was used as an irrelevant control for mAb 246D, and human mAb 447 (IgG3) to the V3 loop of HIV-1 gp120 (Conley et al., 1994) was used as a positive control for the FACS studies. B) PBMCs treated with anti-gp41 ²¹³B1-mAb. The PBMCs exposed to HIV-1 are referred to as "infected" cells and those which were not exposed to the virus as "non-infected" cells. Note sparing of non-infected cells.

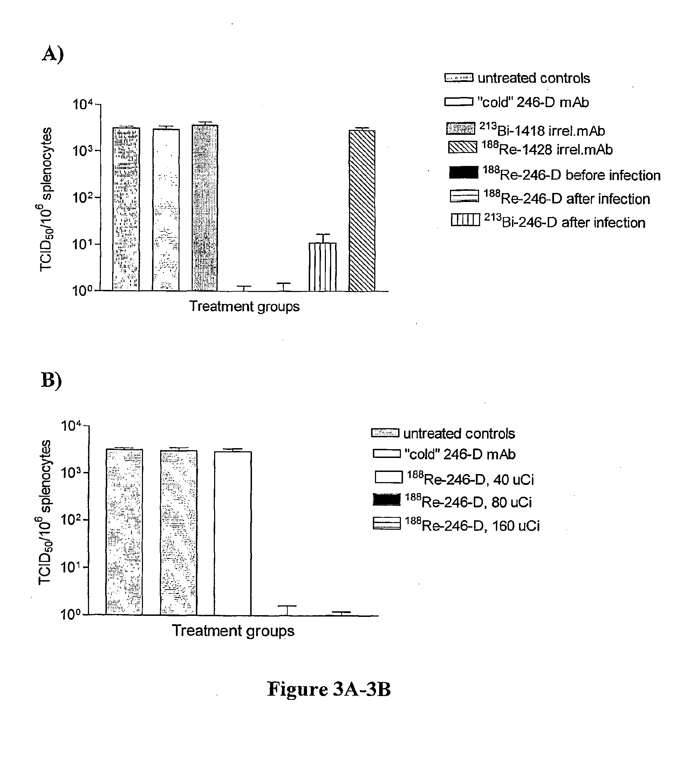

Figure 3A-3B. RIT of SCID mice infected intrasplenically with JR-CSF-infected human PBMCs with ¹⁸⁸Re- and ²¹³Bi-labeled human anti-gp41 mAb 246-D. A) mice received either 20 µg "cold" anti-gp41 mAb 246-D, 100 µCi (20 µg) ²¹³Bi-1418 or 80 µCi (20 µg) ¹⁸⁸Re-1418 as isotype-matching controls, 80 µCi (20 µg) ¹⁸⁸Re-246-D, or 100 µCi (20 µg) ²¹³Bi-246-D IP 1 hour after infection with PBMCs. In some experiments mice were given 80 µCi (20 µg) ¹⁸⁸Re-246-D IP 1 h prior to infection with PBMCs. B) Mice were given 40, 80 or 160 µCi (20 µg) ¹⁸⁸Re-246-D IP, 20 µg "cold" mAb 246-D or left untreated.

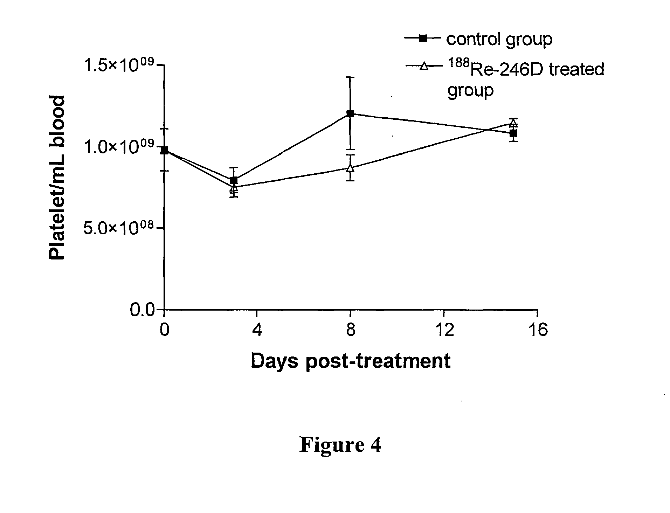

Figure 4. Lack of hematological toxicity of RIT of SCID mice infected intrasplenically with JR-CSF-infected human PBMCs. Platelet counts in the blood of SCID mice injected intrasplenically with HIV-1-infected hPBMCs and either treated with 160 µCi (20 µg) ¹⁸⁸Re-246-D IP 1 hour after infection with PBMCs or untreated. Blood was collected from the tail vein on days 0, 4, 8 and 15 days post-therapy.

### DETAILED DESCRIPTION OF THE INVENTION

The subject invention is directed to a method for treating a subject infected with human immunodeficiency virus (HIV) which comprises administering to the subject an amount of a radiolabeled antibody effective to kill HIV infected cells, wherein the antibody is specific for a HIV envelope antigen (protein or polysaccharide) and wherein the radiolabeled antibody specifically binds to cells that are infected with HIV virus and that express the HIV envelope antigen (protein or polysaccharide) to which the antibody specifically binds.

As used herein, the term "treat" a subject with an HIV infection means to kill cells within the subject that contain HIV, to reduce the number of HIV particles causing the infection in the subject, to prevent the HIV infection from spreading in the subject, to reduce the further spread of HIV infection in the subject, to prevent the establishment of HIV infection in the subject, to treat the HIV infection, to improve symptoms associated with HIV infection, to reduce or prevent opportunistic infection associated with HIV infection, and/or to eliminate the HIV infection. The treatments disclosed herein are also expected to reduce the likelihood of spread of HIV infection to new subjects.

The invention also provides a pharmaceutical composition formulated in dosage form, comprising a radiolabeled antibody and a pharmaceutically acceptable carrier, wherein the antibody is specific for a HIV envelope protein and wherein the dosage is appropriate to kill cells infected with HIV in a subject.

The subject can be any animal that is infected with HIV and is preferably a human.

The invention also provides a method for killing cells infected with human immunodeficiency virus (HIV) which comprises contacting the cells with an amount of a radiolabeled antibody effective to kill HIV infected cells, wherein the antibody is specific for a HIV envelope antigen (protein or polysaccharide) and wherein the radiolabeled antibody specifically binds to cells that are infected with HIV virus and that express the HIV envelope antigen (protein or polysaccharide) to which the antibody specifically binds.

As used herein, the term "antibody" encompasses whole antibodies and fragments of whole antibodies wherein the fragments specifically bind to a HIV envelope protein. Antibody fragments include, but are not limited to, F(ab')₂ and Fab' fragments and single chain antibodies. F(ab')₂ is an antigen binding fragment of an antibody molecule with deleted crystallizable fragment (Fe) region and preserved binding region. Fab' is 1/2 of the F(ab')₂ molecule possessing only 1/2 of the binding region. The term antibody is further meant to encompass polyclonal antibodies and monoclonal antibodies. The antibody can be, e.g., a neutralizing antibody or a non-neutralizing antibody. Preferably, the antibody is a non-neutralizing antibody, since neutralizing antibodies often bind to highly variable motifs in viral antigens that are vulnerable to antigenic variation..

The antibody can be, e.g., any of an IgA, IgD, IgE, IgG, or IgM antibody. The IgA antibody can be, e.g., an IgA1 or an IgA2 antibody. The IgG antibody can be, e.g., an IgG1, IgG2, IgG2a, IgG2b, IgG3 or IgG4 antibody. A combination of any of these antibodies subtypes can also be used. One consideration in selecting the type of antibody to be used is the desired serum half-life of the antibody. IgG has a serum half-life of 23 days, IgA 6 days, IgM 5 days, IgD 3 days, and IgE 2 days (Abbas et al., 2000). Another consideration is the size of the antibody. For example, the size of IgG is smaller than that of IgM allowing for greater penetration of IgG into tissues. IgA, IgG, and IgM are preferred antibodies.

The antibody can be specific for any HIV envelope protein, e.g. glycoprotein gp120, gp41 or gp160. Glycoprotein gp160 is a precursor polypeptide, which when cleaved forms gp120 and gp41 (e.g., Kibler et al., 2004). The antibody can target protein or polysaccharide epitopes. Combinations of different antibodies can be used, where each different antibody binds to a different epitope. The HIV can be any subtype of HIV, e.g. HIV type 1 or HIV type 2. HIV type 1 induces AIDS. HIV type 2 also leads to immune suppression; however, HIV-2 is not as virulent as HIV-1. Numerous antibodies that bind to a HIV envelope protein have been described (e.g., Gorny and Zolla-Pazner, 2000; Nádas et al., 2004; Nyambi et al., 2000; Pincus et al., 2003; Till et al., 1989; Xu et al., 1991; Zolla-Pazner, 2004; U.S. Patent Nos. 5,731,189, 6,241,986 and 6,395,275).

The antibody is preferably a human antibody. However, the antibody can be a non-human antibody such as a goat antibody or a mouse antibody. Non-human antibodies can be used in subjects infected with HIV due to the immune system suppression that occurs in HIV infected subjects. In fact, the molecule carrying the radioactive isotope need not be immunoglobulin since all that is required is a molecule with specificity for binding to a viral antigen expressed on a virally infected cell. Although such molecules are usually proteins, there is no exclusionary requirement for this type of compound and it is conceivable that polysaccharides, lipids, and even small synthetic molecules can be designed to deliver targeted cytotoxic radiation.

Antibodies can be "humanized" using standard recombinant DNA techniques. By transferring the mouse antibody binding site coding region into a human antibody gene, a "human antibody" can be engineered which retains the specificity and biological effects of the original mouse antibody but has the potential to be nonimmunogenic in humans. Additionally, antibody effector functions can be improved through manipulation of the antibody constant region genes (e.g., Clark, 2000; Jolliffe, 1993; LoBuglio et al., 1989). Humanized monoclonal antibodies to gp120 have been described (Dezube et al., 2004; Major et al., 1994). An anti-gp120 humanized monoclonal antibody has been shown to be well tolerated in human subjects in a phase I study (Dezube et al., 2004).

The invention also describes using a radiolabeled agent effective to kill HIV infected cells, wherein the agent is specific for a HIV envelope antigen and wherein the radiolabeled agent specifically binds to cells that are infected with HIV virus and that express the HIV envelope antigen to which the agent specifically binds. The chemical composition of the antigen can be, e.g., protein or polysaccharide. Examples of agents that bind to HIV envelope antigens include peptides and aptamers. The agent can be, e.g., a neutralizing agent or a non-neutralizing agent. Preferably, the agent is a non-neutralizing agent.

Examples of HIV envelope glycoprotein-binding peptides include Fuzeon® and retrocyclin-1. Fuzeon® (also known as T-20 or enfuvirtide) is a C-peptide derived from the gp41 C-terminal heptad repeat (CHR) region and is the first member of a new class of anti-HIV drugs known as HIV fusion inhibitors. T-20 may inhibit HIV-1 entry by targeting multiple sites in gp41 and gp120 (Liu et al., 2005). Retrocyclin-1 is a theta-defensin peptide which binds to gp120 (Owen et al., 2004). Neutralizing (Khati et al., 2003) and non-neutralizing (Sayer et al., 2002) aptamers that bind to gp120 have been described. A neutralizing antibody or agent is one that reacts with a HIV envelope protein and destroys or inhibits the infectivity and/or virulence of the HIV virus. Methods for generating peptides (Valadon et al., 1996) and aptamers (U.S. Patent No. 5,756,291) have been described.

The antibody could also target an antigen that is expressed in HIV-infected cells, but not in non-HIV-infected cells, where the antigen may have viral, mammalian, or combined origin.

Apart from cost and availability, two characteristics are important in the choice of a radioisotope - emission range in the tissue and half-life. Preferably, the antibody or agent is radiolabeled with an alpha emitter or a beta emitter. Alpha emitters have a short emission range in comparison to beta emitters. Examples of alpha emitters include 213-Bismuth (half-life 46 minutes), 223-Radium (half-life 11.3 days), 224-Radium (half-life 3.7 days), 225-Radium (half-life 14.8 days), 225-Actinium (half-life 10 days), 212-Lead (half-life 10.6 hours), 212-Bismuth (half-life 60 minutes), 211-Astatine (half-life 7.2 hours), and 255-Fermium (half-life 20 hours). A preferred alpha emitter is ²¹³Bi, which emits a high LET α-particle with E=5.9 MeV with a path length in tissue of 50-80 µm. Theoretically a cell can be killed with one or two α-particle hits. ²¹³Bi is the only α-emitter that is currently available in generator form, which allows transportation of this isotope from the source to clinical centers within the United States and abroad.

Examples of beta emitters include 188-Rhenium (half-life 16.7 hours), 32-Phosphorous (half-life 14.3 days), 47-Scandium (half-life 3.4 days), 67-Copper (half-life 62 hours), 64-Copper (half-life 13 hours), 77-Arsenic (half-life 38.8 hours), 89-Strontium (half-life 51 days), 105-Rhodium (half-life 35 hours), 109-Palladium (half-life 13 hours), 111-Silver (half-life 7.5 days), 131-Iodine (half-life 8 days), 177-Lutetium (half-life 6.7 days), 153-Samarium (half-life 46.7 hours), 159-Gadolinium (half-life 18.6 hours), 186-Rhenium (half-life 3.7 days), 166-Holmium (half-life 26.8 hours), 166-Dysprosium (half-life 81.6 hours), 140-Lantanum (half-life 40.3 hours), 194-Irridium (half-life 19 hours), 198-Gold (half-life 2.7 days), 199-Gold (half-life 3.1 days), 90-Yttrium (half-life 2.7 days), 177-Lutetium (half-life 6.7 days) and 131-Iodine (half-life 8 days). Preferred beta emitters include 131-Iodine, 90-Yttrium, 188-Rhenium, 186-Rhenium, 177-Lutetium, 166-Holmium, 67-Copper, and 64-Copper, with the high-energy β-emitter 188-Rhenium (Eₘₐₓ = 2.12 MeV) being most preferred. ¹⁸⁸Re has the additional advantage that it emits γ-rays which can be used for imaging studies.

The radioisotope can be attached to the antibody or agent using any known means of attachment used in the art, including interactions such as avidin-biotin interactions, "direct" radiolabeling (Dadachova and Mirzadeh, 1997) and radiolabeling through a bifunctional chelating agent (Saha, 1997). Preferably, the radioisotope is attached to the antibody or agent before the radioisotope or the antibody or agent is administered to the subject.

The invention also describes the use of a combination of antibodies and/or agents radiolabeled with different radiolabels. Preferably, the radioisotopes are isotopes of a plurality of different elements. In a preferred embodiment, at least one radioisotope in the plurality of different radioisotopes is a long range (beta) emitter and at least one radioisotope is a short range (alpha) emitter. Preferably, the beta emitter is 188-Rhenium. Preferably, the alpha emitter is 213-Bismuth.

It is known from radioimmunotherapy studies of tumors that whole antibodies usually require from 1 to 3 days time in circulation to achieve maximum targeting. While slow targeting may not impose a problem for radioisotopes with relatively long half-lives such as ¹⁸⁸Re (t_{1/2}=16.7 hours), faster delivery vehicles may be preferred for short-lived radioisotopes such as ²¹³Bi (t_{1/2}=46 min). The smaller F(ab')₂ and Fab' fragments or domain-deleted antibodies provide much faster targeting which matches the half-lives of short-lived radionuclides (Milenic, 2000; Buchsbaum, 2000). A 'domain-deleted' antibody is an anitbody from which a particular domain, e.g. CH2, has been deleted and replaced with a peptide linker for the purpose of optimizing its therapeutic potential (Milenic, 2000).

In order to calculate the dose of the radioisotope which can significantly decrease or eliminate infection burden without radiotoxicity to vital organs, a diagnostic scan of the patient with the antibody or agent radiolabeled with diagnostic radioisotope or with low activity therapeutic radioisotope can be performed prior to therapy, as is customary in nuclear medicine. The dosimetry calculations can be performed using the data from the diagnostic scan (Early and Sodee, 1995).

Clinical data (Sgouros et al., 1999; Paganelli et al., 1999) indicate that fractionated doses of radiolabeled antibodies are more effective than single doses against tumors and are less radiotoxic to normal organs. Depending on the status of a patient and the effectiveness of the first treatment with RIT, the treatment may consist of one dose or several subsequent fractionated doses.

The dose of the radioisotope for humans will typically be between about 1-500 mCi.

The radiolabeled antibody can be delivered to the subject by a variety of means. Preferably, the radiolabeled antibody or agent is administered parenterally. The radiolabeled antibody can be injected, for example, into the bloodstream, into a muscle or into an organ such as the spleen.

The HIV-infected cell that is targeted and killed by the radiolabeled antibody or agent can be any of, e.g., but not limited to, a lymphocyte, such as a T lymphocyte or a CD4⁺ T lymphocyte, a monocyte, a macrophage, an astrocyte and/or a microglial cell.

Despite the effectiveness of the radiolabeled antibodies in killing cells infected with HIV, the radiolabeled antibody does not kill more than 50% of free HIV virus particles *in vitro* in a solution containing free HIV particles. Typically, no killing of free viral particles can be detected under *in vitro* conditions.

The invention also provides a method of making a composition effective to treat a subject infected with HIV which comprises admixing a radiolabeled antibody and a carrier, wherein the antibody or agent specifically binds to a HIV envelope protein and is effective to kill HIV-infected cells.

As used herein, the term "carrier" encompasses any of the standard pharmaceutical carriers, such as a sterile isotonic saline, phosphate buffered saline solution, water, and emulsions, such as an oil/water or water/oil emulsions.

The invention further provides for the use of a radiolabeled antibody for the preparation of a composition for treating a subject infected with human immunodeficiency virus (HIV), wherein the antibody is specific for a HIV envelope protein and wherein the radiolabeled antibody specifically binds to cells that are infected with HIV virus and that express the HIV envelope protein to which the antibody specifically binds.

The methods of treatment described herein can be used in combination with other therapies against HIV (e.g., Hamer, 2004). For example, agents that induce transcription of latent provirus can be used to express viral proteins in latently infected resting CD4 T cells. HAART therapy can be used to prevent the spread of infection by virus released from cells killed by radioimmunotherapy.

This invention will be better understood from the Experimental Details, which follow. However, one skilled in the art will readily appreciate that the specific methods and results discussed are merely illustrative of the invention as described more fully in the claims which follow thereafter.

### EXPERIMENTAL DETAILS

### Materials and Methods

*Antibodies.* Goat polyclonal antibody (Ab) against gp-120 (IgGl) was purchased from Biodesign International (Saco, ME). Murine 18B7 monoclonal antibody (mAb) (IgG1) specific for cryptococcal polysaccharide (Casadevall et al., 1998) was used as an isotype-matching control. Human anti-gp41 (cluster I) mAb 246D was obtained from Dr. Susan Zolla-Pazner, New York University School of Medicine. The 246 D antibody was produced as described in Nyambi et al. (2000a). The 246 D antibody was previously described in publications by Dr. Zolla-Pazner and her colleagues (Gorny and Zolla-Pazner, 2000; Nyambi et al., 2000a; Robinson et al., 1991; Xu et al., 1991; U.S. Patent No. 5,731,189). As described in U.S. Patent No. 5,731,189, lymphoblastoid cell line 126-6 producing human monoclonal antibodies directed against gp41 was deposited with the American Type Culture Collection (10801 University Boulevard, Manassas, Virginia 21110-2209) on February 24, 1989 and received ATCC Accession number CRL 10037. Human mAb 1418 (IgG1) to parvovirus B19 (Gigler et al., 1999) was used as an irrelevant control for mAb 246D, and human mAb 447 (IgG3) to the V3 loop of HIV-1 gp120 (Conley et al., 1994) was used as a positive control in the FACS studies. Prior to use the antibodies were purified by affinity chromatography.

*Radioisotopes and quantification of radioactivity.* ¹⁸⁸Re in the form of Na perrhenate (Na¹⁸⁸ReO₄) was eluted from a ¹⁸⁸W/¹⁸⁸Re generator (Oak Ridge National Laboratory (ORNL), Oak Ridge, TN). Actinium-225 (²²⁵Ac) for construction of a ²²⁵ Ac/²¹³Bi generator was acquired from the Institute for Transuranium Elements, Karlsruhe, Germany. The ²²⁵Ac/²¹³Bi generator was constructed using MP-50 cation exchange resin, and ²¹³Bi was eluted with 0.15 M HI (hydroiodic acid) in the form of ²¹³BiI₅²⁻ as described in Boll et al. (1997). A gamma counter (Wallac) with an open window was used to count the ¹⁸⁸Re and ²¹³Bi samples.

*Radiolabeling of antibodies with ¹⁸⁸Re and ²¹³Bi.* Antibodies were radiolabeled with beta-emitter ¹⁸⁸Re (half-life 17.0 h) or alpha-emitter ²¹³Bi (half-life 45.6 min). Abs were labeled "directly" with ¹⁸⁸Re via reduction of antibody disulfide bonds by incubating the antibody with 75-fold molar excess of dithiothreitol (Dadachova and Mirzadeh, 1997) for 40 min at 37°C followed by centrifugal purification on Centricon-30 or -50 microconcentrators with 0.15 M NH₄OAc, pH 6.5. Simultaneously 3-10 mCi (110-370 MBq) ¹⁸⁸ReO₄- in saline was reduced with SnCl₂ by incubation in the presence of Na gluconate, combined with purified reduced antibodies and kept at 37°C for 60 min. Radioactivity not bound to the antibody was removed by centrifugal purification on Centricon microconcentrators.

For radiolabeling with ²¹³Bi, Abs were conjugated to bifunctional chelator N-[2-amino-3-(p-isotluocyanatophenyl)propyl]-trans-cyclohexane-1,2-diamine-N, N', N", N"', N""-pentaacetic acid (CHXA") as in Boll et al., 1997, Chappell et al., 2000, Dadachova et al., 1997, and Mirzadeh et al., 1990. The average final number of chelates per antibody molecule was determined by the Yttrium-Arsenazo III spectrophotometric method (Pippin et al., 1992). CHXA"-conjugated Abs were radiolabeled with ²¹³Bi by incubating them for 5 min with ²¹³BiI₅²⁻ at room temperature. If required, the radiolabeled antibodies were purified by size exclusion HPLC (TSK-Gel^{®} G3000SW, TosoHaas, Japan).

*In vitro killing of ACH-2 cells.* An ACH-2 cell line, a latent T-cell clone infected with HIV-IIIB that produces steady low levels of supernatant RT and p24, was obtained through the NIH AIDS Research and Reference Reagent Program, Division of AIDS, NIAID, NIH: ACH-2, catalogue #349 from Dr. Thomas Folks. HIV-1 chronically infected human T-cells ACH-2 (phytohaemagglutinin (PHA)-stimulated, phorbol myristate (PMA)-stimulated, and non-stimulated) were treated with 0-50 µCi of ¹⁸⁸Re-labeled Abs, 0-20 µCi ²¹³Bi-labeled Abs or with matching amounts (2.5-12.5 µg) of "cold" Abs. Approximately 2×10⁵ cells per sample were used. The cells were incubated with radiolabeled or "cold" Abs at 37°C for 3h, transferred into fresh cell culture medium and then incubated in 5% CO₂ at 37°C for 72 h. The number of viable cells 72 h post-treatment was assessed using blue dye exclusion assay.

*Treatment of HIV1-infected and non-infected peripheral blood mononuclear cells (PBMCs) with radiolabeled mAbs.* Human Peripheral Blood Mononuclear Cells (PBMCs) obtained from the New York Blood Center (NY, NY) were stimulated with PHA and interleukin-2 (IL-2) 48 h prior to infection with HIV-1 strain JR-CSF (obtained through the NIH AIDS Research and Reference Reagent Program, Division of AIDS, NIAID, NIH: HIV-1_{JR-CSF}, catalogue #394 from Dr. Irvin S.Y. Chen). While the number of ACH-2 cells infected with HIV-1 was almost 100%, only a fraction (~10-30%) of the PBMCs were infected with HIV-1 as determined by limiting dilution co-culture technique (Ho et al., 1989). Cells exposed to HIV-1 are referred to as "infected" cells and those which were not exposed to the virus are referred to as "non-infected" cells. At 48 h after infection with HIV-1, infected PBMCs were treated with 0-20 µCi ²¹³Bi-labeled Abs or with matching amounts (2.5-12.5 µg) of "cold" Abs. Approximately 2x10⁵ cells per sample were used. As controls, non-infected PBMCs were treated with ²¹³Bi-anti-gp41 mAb. The cells were incubated with radiolabeled or "cold" Abs at 37°C for 3h, transferred into fresh cell culture medium and then incubated in 5% CO₂ at 37°C for 72 h. The number of viable cells 72 h post-treatment was assessed using blue dye exclusion assay.

*Flow cytometric analysis of mAbs binding to virus infected cells.* Binding studies of human mAbs to the surface of hPBMCs infected with the JR-CSF strain of HIV-1 were performed as described previously (Zolla-Pazner et al., 1995). Briefly, PHA-stimulated hPBMCs were infected with 1 ml of stock HIV-1_{JR-CSF} virus and cultured for 13 days in medium supplemented with human recombinant IL-2 (20 U/ml, Boehringer Mannheim Biochemicals, Indianopolis, IN). The cells were incubated with each human mAb at 10 µg/ml for 1 h on ice, washed and reincubated with PE-labeled goat F(ab')₂ anti-human IgG(γ) (Caltag Laboratories, Burlingame, CA). Using a FACScan flow cytometer (Becton Dickinson), live lymphocytes were selected for analysis by gating with forward and 90° scatter. The negative control consisted of cells from infected cultures stained with the conjugated anti-IgG in the absence of a human mAb.

*Treatment of HIV infected PBMCs pre-incubated with HIV positive blood* Human PBMCs were grown and infected with JR-CSF strain of HIV1 as described above. 2×10⁵ infected PBMCs were incubated for 1 h at 37°C with 200 µL of undiluted serum from a HIV 1-positive patient, or with the same volume of 1:10 or 1:100 diluted HIV 1-positive serum using HIV-negative serum as a diluent, or with HIV-negative serum only. Following the incubation the cells were washed with PBS, 1 mL PBS per sample was added and the cells were treated with 20 µCi ²¹³Bi-anti-gp41 mAb (~12.5 µg) or left untreated. The cells were incubated with radiolabeled mAb at 37°C for 3 h, transferred into fresh cell culture medium and then incubated in 5% CO₂ at 37°C for 72 h. The number of viable cells 72 h post-treatment was assessed using blue dye exclusion assay.

*Treatment of naked HIV1 virus with radiolabeled anti-gp41 mAb*. Viral particles were incubated with mAbs for 3 h, followed by infection of healthy PBMCs. On Day 6 post-infection the cultures were analyzed for the presence of HIV core protein p24 by core Profile ELISA (DuPont-NEN).

*Determination of splenic uptake of radiolabeled mAbs.* Two groups of SCID mice were used in this experiment. One group was injected intrasplenically with HIV-1 infected PBMCs and the second group was injected with non-infected PBMCs (25 million cells per mouse). One hour later 20 µCi (20 µg) ¹⁸⁸Re-246-D mAb was given IP to each mouse. Three hours post-injection the animals were sacrificed, their spleens removed, blotted from blood, weighed, counted in a gamma counter, and the percentage of injected dose per gram (ID/g) was calculated.

*Determination of platelet counts in mice treated with radiolabeled mAbs.* Platelet counts were used as a marker of RIT toxicity in treated animals. For measurement of platelet counts, the blood of SCID mice injected intrasplenically with HIV-1-infected hPBMCs and either treated with 100 µCi (20 µg) ²¹³Bi-246-D or 160 µCi (20 µg) ¹⁸⁸Re-246-D IP 1 hour after infection with PBMCs or untreated was collected from the tail vein into 200 µL 1% ammonium oxalate on day 0, 4, 8 and 15 days post-therapy. Platelets were counted in a hemocytometer, using phase contrast, at 400 times magnification, as described in Miale (1982).

*Treatment of HIV1-infected mice with radiolabeled mAbs.* Human PBMCs were stimulated with PHA and IL-2 48 h prior to infection with HIV-1 strain JR-CSF. At 48 h after infection with HIV-1, infected PBMCs were injected intrasplenically (25 million cell per animal) into groups of SCID mice (10 mice per group). Mice received either 20 µg "cold" anti-gp41 mAb 246D, 100 µCi (20 µg) ²¹³Bi-1418 or 80 µCi (20 µg) ¹⁸⁸Re-1418 as isotype-matching controls, 80 µCi (20 µg) ¹⁸⁸Re-246D, or 100 µCi (20 µg) ²¹³Bi-246D IP 1 hour after infection with PBMCs. In some experiments mice were given 80 µCi (20 µg) ¹⁸⁸Re-246D IP 1 h prior to infection with PBMCs. The SCID mice were sacrificed 72 hours after treatment and the spleens were harvested and processed. A limiting dilution co-culture of the splenocytes was performed using freshly activated PBMCs as described in Wang et al. (2002). Supernatants were harvested on day 8 after initiation of co-culture and analyzed for the presence of HIV-1 core protein p24 by core Profile ELISA (DuPont-NEN). Data are reported as infected splenocytes/10⁶ splenocytes. The number of HIV-1-infected cells present in the spleen was measured using limiting dilution quantitative co-culture as described by Ho et al. (1989). This technique measures the number of cells capable of producing infectious HIV-1. Five-fold dilutions of cells isolated from each spleen (in the range 1 × 10⁶ - 3.2 × 10² cells) were cultured in duplicate at 37°C in 24-well culture plates with PHA-activated hPBMCs (1 × 10⁶ cells) in 2.0 mL of RPMI 1640 medium containing fetal calf serum (10% vol/vol) and interleukin-2 (32 U/mL). The HIV-1 p24 antigen content of the supernatant was measured 1 week later, using the HIV-1 p24 core profile ELISA (DuPont-NEN). The lowest number of added cells that infected at least half the duplicate cultures with HIV-1 was determined and represented the frequency of cells productively infected with HIV-1 in each spleen, reported as TCID₅₀/10⁶ splenocytes. In dose response experiment the groups of infected mice were given 40, 80 or 160 µCi (20 µg) ¹⁸⁸Re-246-D IP, 20 µg "cold" mAb 246-D or left untreated and the efficacy of the therapy was assessed.

*Statistical analysis.* Prism software (GraphPad, San Diego, CA) was used for statistical analysis of the data. Student's t-test for unpaired data was employed to analyze differences in the number of viable ACH-2 cells, PBMCs or infected splenocytes/10⁶ splenocytes between differently treated groups during *in vitro* and *in vivo* therapy studies, respectively. Differences were considered statistically significant when P values were < 0.05.

### Results

*In vitro killing of HIV-infected ACH-2 cells with radiolabeled mAbs.* To determine the capacity of RIT to kill HIV-1 infected cells, goat polyclonal anti-gp120 Ab was labeled with radioisotopes with distinctly different emission characteristics - 213-Bismuth (²¹³Bi, a radioisotope that emits alpha-particles which are He atoms with the charge of +2 and mass of 4) and 188-Rhenium (¹⁸⁸Re, a radioisotope that emits high energy beta-particles (electrons)). ²¹³Bi and ¹⁸⁸Re have different emission ranges in tissue - 50-80 µm for ²¹³Bi versus 10 mm (average) for ¹⁸⁸Re. Both radioisotopes have been used in pre-clinical and clinical settings.

HIV-1-infected ACH-2 cells were incubated with ¹⁸⁸Re-anti-gp120 Ab, ¹⁸⁸Re-control Ab (irrelevant murine mAb 18B7) or "cold" anti-gp120 Ab. Significant killing of HIV-infected ACH-2 cells was observed with ¹⁸⁸Re-anti-gp120 Ab (Figure 1A). In contrast, the control Ab ¹⁸⁸Re-18B7 with the same specific activity produced only minimal killing within the investigated range of activities (P=0.01). The significantly higher killing associated with the specific antibody almost certainly reflects higher radiation exposure for ACH-2 cells as a consequence of Ab binding to gp120 glycoprotein expressed on the surface of ACH-2 cells. No killing of ACH-2 cells was observed for non-radiolabeled ("cold") anti-gp120 Ab. This study established the feasibility of targeting viral proteins in chronically infected cells with RIT. However, a significant percentage of cells survived co-incubation with the ¹⁸⁸Re-labeled antibody. The inability of the ¹⁸⁸Re-anti-gp120 Ab to completely eliminate the cells *in vitro* reflects the fact that the range of ¹⁸⁸Re-emitted beta-particles is several mm which results in most of the radiation passing through the cells layer without scoring lethal hits on the cells - a well-documented effect observed for *in vitro* RIT experiments with cancer cell lines and Abs labeled with beta-emitters.

Since the relative biological effectiveness (RBE) of α-particles is significantly higher than that of β particles by virtue of their mass, charge and energy (Casarett, 1968, Wheldon 1994), the study was repeated using 2.5 times lower radioactivity in the incubation of ACH-2 cells with ²¹³Bi-labeled antibodies than in ¹⁸⁸Re experiments. Five µCi ²¹³Bi-anti-gp120 per 2×10⁵ ACH-2 cells eradicated virtually 100% of the cells (Figure 1B). The cytocidal activity of the irrelevant mAb ²¹³Bi-18B7 was negligible (P = 0.0004) at the activity concentrations studied. This result attests to a very high killing efficiency of ²¹³Bi towards HIV-infected ACH-2 cells, as high linear energy transfer (LET) of α-particles makes it possible to kill a cell with 1-2 hits, whereas several hundred hits per cell are needed when β-emitting radionuclides are used. The results observed *in vitro* warranted the testing of RIT for elimination of HIV-infected cells *in vivo.*

*In vitro killing of HIV-1 infected human PBMCs with radiolabeled mAb.* Human anti-gp41 mAb 246-D was used for these studies. This mAb binds specifically to HIV-1-infected cells as demonstrated by flow cytometry of hPBMCs infected with HIV-1_{JR-CSF} (Figure 2A). Mab 246-D does not bind to the 6-helical bundle resulting from CD4 activation but rather to an epitope near the disulfide loop of gp41 which is an immunodominant epitope (Gorny et al., 2000), and is broadly cross-reactive with genetically diverse HIV-1 strains (Nyambi et al., 2000a,b). After anti-gp41 mAb 246-D was linked with ²¹³Bi, it was very effective in eliminating HIV-1-infected hPBMCs when compared to "cold" 246-D and a ²¹³Bi-radiolabeled control mAb (Figure 2B). The number of hPBMCs killed by mAb ²¹³Bi-246-D was greater than the number of infected cells. This most likely reflects a "cross-fire" effect from alpha-particles emanating from adjacent HIV-1-infected cells that contain bound ²¹³Bi-mAb in the setting of cell crowding at the bottom of tissue culture wells.

*Sparing of PBMCs not infected with HIV-1 following treatment with radiolabeled mAbs.* As shown in Figure 2B, ²¹³Bi-anti-gp41 was effective in killing PBMCs infected with HIV. Importantly, incubation of non-infected PBMCs with mAb ²¹³Bi-246-D produced no significant killing of the cultured cells implying that in the absence of HIV-1 antigens, gp41-specific radiolabeled mAb was non-toxic (Figure 2B).

*Killing of HIV infected PBMCs pre-incubated with HIV positive blood.* Human PBMCs infected with HIV were incubated with serum from a HIV1-positive patient before exposure of the PBMCs to ²¹³Bi-anti-gp41 mAb. 100% killing of the PBMCs was observed following treatment with ²¹³Bi-anti-gp41 mAb (results not shown). Thus, antibodies to gp41 glycoprotein in HIV1-positive serum, which can potentially compete with radiolabeled anti-gp41 mAb, were not able to block the binding of ²¹³Bi-anti-gp41 mAb to the PBMCs which resulted in their killing.

*Sparing of naked HIV-1 virus following treatment with radiolabeled anti-gp41 mAb.* Table 1 compares the effects of treating HIV-1 viral particles with radiolabeled ("hot") anti-gp41 Ab, cold anti-gp41, and hot irrelevant mAb. Radiolabeled Abs to the gp41 HIV envelope protein were not effective in killing HIV viral particles.

*Elimination of HIV-1 infected PBMCs in mice by RIT.* Human anti-gp41 mAb 246D was used for *in vivo* experiments. Targeting gp41 has the advantage that this protein is reliably expressed on the surface of chronically infected cells. In addition to the advantages of using human mAb relative to goat polyclonal sera with regards to specific activity and specificity, published data indicate that immunotoxins are more efficient against HIV-infected cells when delivered to the cells by anti-gp41 mAbs rather than anti-gp120 mAbs (Pincus et al., 2003). In the present mouse model, HIV-infected cells are residing in the spleen, which is one of the significant reservoirs of HIV-harboring cells in humans, and thus this model has advantages over more artificial lymphoma tumor-type models (Pincus et al., 2003).

Human PBMCs infected with HIV-1_{JR-CSF} were injected into the spleens of SCID mice and the mice were treated as indicated. Doses of 80 µCi dose ¹⁸⁸Re-labeled 246-D and 100 µCi ²¹³Bi-246-D were chosen because these doses were therapeutic and safe in experimental RIT of fungal and bacterial infections (Dadachova et al., 2004a,b). The mice were evaluated 72 hours later for the presence of residual HIV-1-infected cells by quantitative co-culture (Conley et al., 1994). The 72 hour time period was chosen to give sufficient time for ¹⁸⁸Re-labeled mAb to deliver a lethal dose of radioactivity to the cells as the ¹⁸⁸Re half-life is 16.9 hr and several half-lives are required for a given radionuclide to deliver the dose to the target.

The results of RIT of SCID mice infected intrasplenically with JR-CSF-harboring human PBMCs are presented in Figure 3. Treatment of infected mice with ¹⁸⁸Re-labeled human anti-gp41 mAb 246D administered either after or before HIV infection of animals significantly reduced the number of HIV-infected cells (Figure 3A). Treatment with ²¹³Bi-246D effectively reduced the number of infected splenocytes by 300-fold (Figure 3A). In contrast, the administration of the matching amounts of "cold" 246D and of radiolabeled irrelevant control antibody 141 did not result in any reduction of the average number of infected cells in the SCID mice spleens (Figure 3A).

¹⁸⁸Re-246-D was more effective *in vivo* than ²¹³Bi-246-D due to the longer physical half-life of ¹⁸⁸Re (16.9 hours versus 46 minutes) allowing the labeled mAb to reach infected cells while still carrying high activity "payload". To investigate the dose-response effect, the mice were treated with 40, 80 and 160 µCi ¹⁸⁸Re-246-D, corresponding to 50, 100 and 200% of the therapeutic dose, respectively. While 40 µCi ¹⁸⁸Re-246-D was not effective in killing infected PBMCs *in vivo,* 160 µCi dose essentially eliminated infected cells (Figure 3B). These results establish that RIT can effectively target and kill HIV-1-infected human PBMCs *in vivo.*

To further investigate the specificity of radiolabeled mAb binding to gp41 HIV-infected hPBMCs, the splenic uptake of ¹⁸⁸Re-246-D mAb was compared in mice injected intrasplenically with hPBMCs and HIV-1 infected hPBMCs. The uptake expressed as percentage of injected dose (ID) per gram of spleen was 8±4 and 57±10% ID/g (P<0.001) for non-infected and infected PBMCs, respectively. This result establishes *in vivo* targeting of ¹⁸⁸Re-246-D to HIV-1-infected cells.

*Lack of hematological toxicity of RIT of HIV infection.* The hematological toxicity of radiolabeled 246-D mAb during HIV-1 infection was evaluated in the SCID mice by platelet counts. The platelet count nadir usually occurs 1 week after radiolabeled antibody administration to tumor-bearing animals (Behr et al., 1999; Sharkey et al., 1997). No changes were observed in platelet counts in mice treated with 100 µCi ²¹³Bi-246D mAb on days 4, 8 and 15 post-treatment in comparison to non-treated infected controls, with platelet counts being stable at (1.5±0.2) × 10⁹ platelet/mL blood (data not shown). For mice given 160 µCi ¹⁸⁸Re-246-D (the highest dose used in this study), a slight drop in platelet count was noted on day 7 post-treatment with counts returning to normal by day 15 (Figure 4). This lack of hematologic toxicity can be explained by the very specific targeting of infected PBMCs by radiolabeled mAb, since gp41 antigen is only expressed on infected cells in the mouse.

**Table 1. Treatment of naked HIV-1 virus with radiolabeled anti-gp41 mAb.**

| Dilution of Virus | 10 | 100 | 1000 | 10,000 | 100,000 |
|---|---|---|---|---|---|
| Cold anti-gp41 | 94.5 | 14.1 | 3.1 | 2.1 | 4.8 |
| Hot irrelevant 1418 mAb | 96.4 | 29.1 | 2.9 | 1.9 | 2 |
| Hot anti-gp41 | 106.7 | 16 | 3.2 | 2 | 2 |

Data show amount of HIV-1 core protein p24 (pg/ml) present.

### Discussion

The present application discloses the efficacy of radioimmunotherapy (RIT) in treating HIV infection using radiolabeled antibodies directed to HIV envelope proteins. The β-emitter 188-Rhenium (¹⁸⁸Re) and α-particle emitter 213-bismuth (²¹³Bi) were used herein as examples of therapeutic radionuclides for RIT of HIV infection. ¹⁸⁸Re (T½=16.7 h) is a high-energy β-emitter (Eₘₐₓ = 2.12 MeV) and has the additional advantage that it emits γ-rays which can be used for imaging studies. ²¹³Bi (T_{½}=45.6 min) emits a high linear energy transfer (LET) α-particle with E=5.9 MeV with a path length in tissue of 50-80 µm. Theoretically a cell can be killed with one or two α-particle hits.

The results disclosed herein demonstrate that RIT is effective against cells harboring HIV both *in vivo* and *in vitro,* but not against naked HIV particles as tested *in vitro.* In contrast, RIT is efficient against fungal and bacterial pathogens (Dadachova et al., 2003, 2004a-c; U.S. Patent Application Publication No. US 2004/0115203) despite the fact that the track range in tissue of radiation emitted by ²¹³Bi and especially by ¹⁸⁸Re is much longer than a fungal or bacterial cell diameter. The apparent inability of RIT to kill naked HIV virus is probably a combination of the extremely small size of viral particles (nanometer range) and their extreme radioresistance.

RIT has several advantages over an immunotoxin approach for treatment of HIV infection. First, the antibody used for delivery of radiation does not need to be internalized to deliver its toxic payload to the cell, since radiation emitted by radioisotopes is cytotoxic without the need for internalization. Second, not every infected cell in the body needs to be targeted by the antibody as particulate radiation kills neighboring cells via "cross-fire" effect (i.e., radiation emanating from a radiolabeled cell hits adjacent cells). Consistent with this mechanism ¹⁸⁸Re-labeled mAbs were more effective *in vivo* (Figure 3A-3B) than *in vitro* (Figure 1A). *In vitro,* the "cross-fire" radiation is largely deposited on the two dimensional surface represented by the cell layer at the bottom of a tissue culture well; whereas *in vivo* there are many infected cells in the nearby three-dimensional space such that "cross-fire" radiation is more effective. Although crossfire effect could also kill non-infected cells *in vivo*, the short penetration of alpha and beta particles is likely to limit this effect to cells in the immediate proximity of infected cells which may also be infected. Third, in contrast to immunotoxins, radiolabeled human antibodies are unlikely to elicit significant immune responses that would limit subsequent use. Fourth, RIT is a potentially much less toxic treatment than the use of immunotoxins where release of the toxin could result in significant systemic toxicity. Finally, a radioactive atom is much smaller than a toxin and consequently, the molecular weight of a radionuclide-antibody conjugate is significantly less than an immunotoxin-antibody conjugate. Smaller mass means the possibility of killing a greater number of infected cells per weight basis of therapeutic agent. The attractiveness of RIT for HIV-1 infection is further enhanced by the recognition that the long-lived infected cellular targets are often lymphocytes, which are among the most radiosensitive cells in the body.

One of the advantages of using RIT against infections as opposed to cancer is that, in contrast to tumor cells, cells expressing microbial antigens are antigenically very different from host tissues and thus provide the potential for exquisite specificity and low cross-reactivity. A large therapeutic window is available because the therapeutic effect disclosed herein was achieved with activities that were significantly lower than the reported maximum tolerated activity (MTA) for ¹⁸⁸Re (800 µCi for IV injection; Sharkey et al., 1997) and ²¹³Bi-labeled IgGs (in excess of 1,000 µCi when given IP; Milenic et al, 2004).

In the clinic, RIT may be most effective when used in combination with highly active antiretroviral therapy (HAART) (Berger et al., 1998), which blocks virus replication in newly infected cells. An exciting use of RIT combined with HAART would be to prevent HIV infection when administered to individuals within the first days of exposure to HIV. In addition, initial treatment of patients soon after infection may reduce the number of HIV-1-infected cells and thereby reduce viral set-point. Moreover, RIT may be a useful adjunct for protocols designed to "flush out" quiescent, latently infected lymphocytes by the administration of factors that promote HIV replication such as valproic acid (Lehrman et al., 2005). The availability of RIT is envisioned to provide a novel treatment for the eradication of HIV-1 infection.

### REFERENCES

Abbas AK, Lichtman AH, Pober JS. Cellular and Molecular Immunology, 4th edition, W.B. Saunders Co., Philadelphia, 2000.
Behr, T.M. et al. High-linear energy transfer (LET) alpha versus low-LET beta emitters in radioimmunotherapy of solid tumors: therapeutic efficacy and dose-limiting toxicity of 213Bi- versus 90Y-labeled CO17-1A Fab' fragments in a human colonic cancer model. Cancer Res. 59, 2635-2643, 1999.
Bera TK, Kennedy PE, Berger EA, Barbas CF 3rd, Pastan I. Specific killing of HIV-infected lymphocytes by a recombinant immunotoxin directed against the HIV-1 envelope glycoprotein. Mol Med. 4(6):384-91, 1998.
Berger, E.A., Moss, B. & Pastan, I. Reconsidering targeted toxins to eliminate HIV infection: you gotta have HAART. Proc. Natl. Acad. Sci. USA. 95: 11511-11513, 1998.
Boll RA, Mirzadeh S, and Kennel SJ. Optimizations of radiolabeling of immuno-proteins with 213-Bi. Radiochim. Acta 79: 145-149, 1997.
Buchsbaum, D.J. Experimental radioimmunotherapy. Semin. Radiat. Oncol. 10: 156-167, 2000.
Carr, A. Toxicity of antiretroviral therapy and implications for drug development. Nat Rev Drug Discov. 2(8):624-34, 2003.
Casadevall A, Cleare W, Feldmesser M, Glatman-Freedman A, Goldman DL, Kozel TR, Lendvai N, Mukherjee J, Pirofski LA, Rivera J, Rosas AL, Scharff MD, Valadon P, Westin K, Zhong Z. Characterization of a murine monoclonal antibody to Cryptococcus neoformans polysaccharide that is a candidate for human therapeutic studies. Antimicrob. Agents Chemother. 42: 1437-1446, 1998.
Casadevall A, Mukherjee J, and Scharff MD. Monoclonal antibody based ELISAs for cryptococcal polysaccharide. J. Immunol. Methods 154: 27-35, 1992.
Casarett, A.P. Radiation Biology. Prentice-Hall, NJ, USA. 367 pp. 1968.
Chappell LL, Dadachova E, Milenic DE, Garmestani K, Brechbiel MW. Synthesis and Characterization of a Novel Bifunctional Chelating Agent for Lead(II). Conjugation to a Monoclonal Antibody, Radiolabeling with Lead-203 and Serum Stability Determination, Nucl. Med. Biol. 27: 93-100, 2000.
Chun TW, Carruth L, Finzi D, Shen X, DiGiuseppe JA, Taylor H, Hermankova M, Chadwick K, Margolick J, Quinn TC, Kuo YH, Brookmeyer R, Zeiger MA, Barditch-Crovo P, Siliciano RF. Quantification of latent tissue reservoirs and total body viral load in HIV-1 infection. Nature 387(6629):183-8, 1997.
Clark, M. Antibody humanization: a case of the 'Emperor's new clothes'? Immunology Today 21(8): 397-402, 2000.
Conley, A.J. et al Neutralization of primary human immunodeficiency virus type 1 isolates by the broadly reactive anti-V3 monoclonal antibody, 447-52D. J Virol. 68: 6994-7000, 1994.
Dadachova E, Bryan RA, Frenkel A, Zhang T, Apostolidis C, Nosanchuk JS, Nosanchuk JD, Casadevall A. Evaluation of acute hematologic and long-term pulmonary toxicities of radioimmunotherapy of Cryptococcus neoformans infection in murine models. Antimicrob Agents Chemother. 48(3):1004-6, 2004a.
Dadachova E, Bums T, Bryan RA, Apostolidis C, Brechbiel MW, Nosanchuk JD, Casadevall A, Pirofski L. Feasibility of radioimmunotherapy of experimental pneumococcal infection. Antimicrob Agents Chemother. 48(5):1624-9, 2004b.
Dadachova E, Howell RW, Bryan RA, Frenkel A, Nosanchuk JD, Casadevall A. Susceptibility of the human pathogenic fungi Cryptococcus neoformans and Histoplasma capsulatum to gamma-radiation versus radioimmunotherapy with alpha- and beta-emitting radioisotopes. J Nucl Med. 45(2):313-20, 2004c.
Dadachova, E., and Mirzadeh, S. The role of tin in the direct labelling of proteins with rhenium-188. Nucl. Med. Biol. 24: 605-608, 1997.
Dadachova, E., Mirzadeh, S., Smith, S.V., Knapp, F.F., and Hetherington, E.L. Radiolabelling antibodies with 166-Holmium. Appl. Rad. Isotop. 48: 477-481, 1997.
Dadachova E, Nakouzi A, Bryan RA, Casadevall A. Ionizing radiation delivered by specific antibody is therapeutic against a fungal infection. Proc Natl Acad Sci U S A. 100(19):10942-7, 2003 (Epub 2003 Aug 20).
Davey RT Jr, Boenning CM, Herpin BR, Batts DH, Metcalf JA, Wathen L, Cox SR, Polis MA, Kovacs JA, Falloon J, et al. Use of recombinant soluble CD4 Pseudomonas exotoxin, a novel immunotoxin, for treatment of persons infected with human immunodeficiency virus. J Infect Dis.170(5):1180-8, 1994.
Dezube BJ, Doweiko JP, Proper JA, Conway B, Hwang L, Terada M, Leece BA, Ohno T, Mastico RA. Monoclonal antibody hNM01 in HIV-infected patients: a phase I study. J Clin Virol. 31 Suppl 1:S45-7, 2004.
Early P.J. and Sodee D.B. Principles and Practice of Nuclear Medicine, Mosby, 1995.
Gigler A, Dorsch S, Hemauer A, Williams C, Kim S, Young NS, Zolla-Pazner S, Wolf H, Gorny MK, Modrow S. Generation of neutralizing human monoclonal antibodies against parvovirus B19 proteins..J. Virol. 73: 1974-1979, 1999.
Goldman DL, Casadevall A, Zuckier LS. Pharmacokinetics and biodistribution of a monoclonal antibody to Cryptococcus neoformans capsular polysaccharide antigen in a rat model of crypto-coccal meningitis: implications for passive immunotherapy, J. Med. Veterinary Mycol. 35: 271-278, 1997.
Goldstein H, Pettoello-Mantovani M, Bera TK, Pastan IH, Berger EA. Chimeric toxins targeted to the human immunodeficiency virus type 1 envelope glycoprotein augment the in vivo activity of combination antiretroviral therapy in thy/liv-SCID-Hu mice. J Infect Dis. 181(3):921-6,2000.
Gorny MK, Zolla-Pazner S. Recognition by human monoclonal antibodies of free and complexed peptides representing the prefusogenic and fusogenic forms of human immunodeficiency virus type 1 gp41. J Virol. 74(13):6186-92, 2000.
Hamer DH. Can HIV be Cured? Mechanisms of HIV persistence and strategies to combat it. Curr HIV Res. 2(2):99-111, 2004.
Hernigou P, Gras G, Marinello G, Dormont D. Inactivation of HIV by application of heat and radiation: implication in bone banking with irradiated allograft bone. Acta Orthop Scand. 71(5):508-12, 2000.
Ho, D.D., Moudgil, T. & Alam, M. Quantitation of human immunodeficiency virus type 1 in the blood of infected persons. N. Engl. J. Med. 321: 1621-1625, 1989.
Jolliffe LK. Humanized antibodies: enhancing therapeutic utility through antibody engineering. Int Rev Immunol. 10(2-3):241-50, 1993.
Khati M, Schuman M, Ibrahim J, Sattentau Q, Gordon S, James W. Neutralization of infectivity of diverse R5 clinical isolates of human immunodeficiency virus type 1 by gp120-binding 2'F-RNA aptamers. J Virol. 77(23):12692-8, 2003.
Kibler KV, Miyazato A, Yedavalli VS, Dayton AI, Jacobs BL, Dapolito G, Kim SJ, Jeang KT. Polyarginine inhibits gp160 processing by furin and suppresses productive human immunodeficiency virus type 1 infection. J Biol Chem. 279(47):49055-63, 2004; Epub 2004 Sep 14.
Komarova LN, Petin VG, Tkhabisimova MD Recovery of yeast cells after exposure to ionizing radiation and hyperthermia, Radiation Biology. Radioecology (in Russian), 42: 54-59,2002.
Lehrman, G., et al. Depletion of latent HIV-1 infection in vivo: a proof-of-concept study. Lancet 366, 549-555, 2005.
Little, S.J. et al. Antiretroviral-drug resistance among patients recently infected with HIV. N. Engl. J. Med. 347, 385-394, 2002.
Liu S, Lu H, Niu J, Xu Y, Wu S, Jiang S. Different from the HIV fusion inhibitor C34, the anti-HIV drug Fuzeon (T-20) inhibits HIV-1 entry by targeting multiple sites in gp41 and gp120. J Biol Chem. 2005 Mar 25;280(12):11259-73. Epub 2005 Jan 7.
LoBuglio AF, Wheeler RH, Trang J, Haynes A, Rogers K, Harvey EB, Sun L, Ghrayeb J, Khazaeli MB. Mouse/human chimeric monoclonal antibody in man: kinetics and immune response. Proc Natl Acad Sci U S A. 86(11):4220-4, 1989.
Major JG, Liou RS, Sun LK, Yu LM, Starnes SM, Fung MS, Chang TW, Chang NT. Construction and characterization of chimeric and humanized forms of a broadly neutralizing monoclonal antibody to HIV-1. Hum Antibodies Hybridomas 5(1-2):9-17, 1994.
Miale, J.B. Laboratory Medicine Hematology, The CV Mosby Company, St. Louis, MO, p. 864, 1982.
Milenic, D.E. Radioimmunotherapy: designer molecules to potentiate effective therapy. Semin. Radiat. Oncol. 10: 139-155, 2000.
Milenic, D., et al. Radioimmunotherapy of human colon carcinoma xenografts using a 213Bi-labeled domain-deleted humanized monoclonal antibody. Cancer Biother. Radiopharm. 19: 135-147, 2004.
Mironenko NV, Alekhina IA, Zhdanova NN, Bulat SA. Intraspecific variation in gamma-radiation resistance and genomic structure in the filamentous fungus Alternaria alternata: a case study of strains inhabiting Chernobyl reactor no. 4. Ecotoxicol. Environ. Saf. 5:177-187, 2000.
Mirzadeh, S., Brechbiel, M.W., Atcher, R.W., and Gansow, O.A. Radiometal labeling of immunoproteins: covalent linkage of 2-(4-isothiocyanatobenzyl) diethylenetriaminepentaacetic acid ligands to immunoglobulin. Bioconjug. Chem. 1: 59-65, 1990. Nádas A, Zhong P, Burda S, Zekeng L, Urbanski M, Gorny MK, Zolla-Pazner S, Nyambi PN. Defining human immunodeficiency virus (HIV) type 1 immunotypes with six human monoclonal antibodies. AIDS Res Hum Retroviruses. 20(1):55-65, 2004.
Nyambi PN, Mbah HA, Burda S, Williams C, Gorny MK, Nadas A, Zolla-Pazner S. Conserved and exposed epitopes on intact, native, primary human immunodeficiency virus type 1 virions of group M. J Virol. 74(15):7096-7107, 2000a.
Nyambi PN et al. Immunoreactivity of intact virions of human immunodeficiency virus type 1 (HIV-1) reveals the existence of fewer HIV-1 immunotypes than genotypes. J Virol. 74:10670-10680, 2000b.
Paganelli G., Zoboli S., Cremonesi M. et al Receptor-mediated radionuclide therapy with 90-Y-DOTA-D-Phe-Tyr3-Octreotide: Preliminary report in cancer patients. Cancer Biother. Radiopharm. 14: 477-483, 1999.
Persaud D, Zhou Y, Siliciano JM, Siliciano RF. Latency in human immunodeficiency virus type 1 infection: no easy answers. J Virol. 77(3):1659-65, 2003.
Pincus SH, Fang H, Wilkinson RA, Marcotte TK, Robinson JE, Olson WC. In vivo efficacy of anti-glycoprotein 41, but not anti-glycoprotein 120, immunotoxins in a mouse model of HIV infection. J Immunol.170(4):2236-41, 2003.
Owen SM, Rudolph DL, Wang W, Cole AM, Waring AJ, Lal RB, Lehrer RI. RC-101, a retrocyclin-1 analogue with enhanced activity against primary HIV type 1 isolates. AIDS Res Hum Retroviruses 20(11):1157-65, 2004.
Pippin, C.G., Parker, T.A., McMurry, T.J., and Brechbiel, M.W. Spectrophotometric method for the determination of a bifunctional DTPA ligand in DTPA-monoclonal antibody conjugates. Bioconjug. Chem. 3: 342-345, 1992.
Ramachandran RV, Katzenstein DA, Wood R, Batts DH, Merigan TC. Failure of short-term CD4-PE40 infusions to reduce virus load in human immunodeficiency virus-infected persons. J Infect Dis. 170(4):1009-13, 1994.
Robinson WE Jr, Gorny MK, Xu JY, Mitchell WM, Zolla-Pazner S. Two immunodominant domains of gp41 bind antibodies which enhance human immunodeficiency virus type 1 infection in vitro. J Virol. 65(8):4169-76, 1991:
Robinson WE Jr, Kawamura T, Gorny MK, Lake D, Xu JY, Matsumoto Y, Sugano T, Masuho Y, Mitchell WM, Hersh E, et al. Human monoclonal antibodies to the human immunodeficiency virus type 1 (HIV-1) transmembrane glycoprotein gp41 enhance HIV-1 infection in vitro. Proc Natl Acad Sci U S A. 87(8):3185-9, 1990.
Saavedra-Lozano J, Cao Y, Callison J, Sarode R, Sodora D, Edgar J, Hatfield J, Picker L, Peterson D, Ramilo O, Vitetta ES. An anti-CD45RO immunotoxin kills HIV-latently infected cells from individuals on HAART with little effect on CD8 memory. Proc Natl Acad Sci U S A. 101(8):2494-9, 2004.
Saavedra-Lozano J, McCoig C, Xu J, Cao Y, Keiser P, Ghetie V, Siliciano RF, Siliciano JD, Picker LJ, Ramilo O, Vitetta ES. An anti-CD45RO immunotoxin kills latently infected human immunodeficiency virus (HIV) CD4 T cells in the blood of HIV-positive persons. J Infect Dis.185(3):306-14, 2002 (Epub 2002 Jan 08).
Saha GB Fundamentals of Nuclear Pharmacy, Springer, New York, pp.139-143, 1997.
Sayeg JA, Birge AC, Beam CA, Tobias CA. The effects of accelerated carbon nuclei and other radiations on the survival of haploid yeast. II. Biological experiments. Radiat. Res. 10:449-461, 1959.
Sayer N, Ibrahim J, Turner K, Tahiri-Alaoui A, James W. Structural characterization of a 2'F-RNA aptamer that binds a HIV-1 SU glycoprotein, gp120. Biochem Biophys Res Commun. 293(3):924-31, 2002.
Schmid AK, Lidstrom ME. Involvement of Two Putative Alternative Sigma Factors in Stress Response of the Radioresistant Bacterium Deinococcus radiodurans. J. Bacteriol. 184(22): 6182-6189, 2002.
Sgouros, G., Ballangrud, A.M., Jurcic, J.G., McDevitt, M.R., Humm, J.L., Erdi, Y.E., Mehta, B.M., Finn, R.D., Larson, S.M. and Scheinberg, D.A. Pharmacokinetics and dosimetry of an alpha-particle emitter labeled antibody: 213Bi-HuM195 (anti-CD33) in patients with leukemia. J. Nucl. Med. 40: 1935-1946, 1999.
Sharkey, R.M. et al. Selection of radioimmunoconjugates for the therapy of well-established or micrometastatic colon carcinoma. Int. J. Cancer 72, 477-485, 1997.
Shvedenko VI, Kabakova NM, Petin VG A Comparative study of RBE of densely ionizing radiation for various criteria of yeast cell death, Radiation Biology. Radioecology (in Russian), 41: 361-365, 2001.
Till MA, Zolla-Pazner S, Gorny MK, Patton JS, Uhr JW, Vitetta ES. Human immunodeficiency virus-infected T cells and monocytes are killed by monoclonal human anti-gp41 antibodies coupled to ricin A chain. Proc Natl Acad Sci U S A. 86(6):1987-91, 1989.
U.S. Patent Application Publication No. US 2004/0115203 A1, published June 17, 2004, Dadachova E, Casadevall A, Nakouzi A. Methods of applying ionization radiation for therapy of infections.
U.S. Patent No. 5,731,189, issued March 24, 1998, Zolla-Pazner S, Gorny MK. Human monoclonal antibodies to human immunodeficiency virus.
U.S. Patent No. 5,756,291, issued May 26, 1998, Griffin L et al., Aptamers specific for biomolecules and methods of making.
U.S. Patent No. 6,241,986, issued June 5, 2001, Zolla-Pazner S, Gorny MK, Karwowska S, Buchbinder A. Human monoclonal antibodies to the CD4-binding domain of HIV, uses thereof and synergistic neutralization of HIV.
U.S. Patent No. 6,395,275, issued May 28, 2002, Barbas CF, Burton DR, Lerner RA. Synthetic neutralizing human monoclonal antibodies to human immumodeficiency virus. Valadon, P., G. Nussbaum, L. F. Boyd, D. H. Margulies, and M. D. Scharff. Peptide libraries define the fine specificity of anti-polysaccharide antibodies to Cryptococcus neoformans. J. Mol. Biol. 261:11-22, 1996.
Wang EJ, Pettoello-Mantovani M, Anderson CM, Osiecki K, Moskowitz D, Goldstein H. Development of a novel transgenic mouse/SCID-hu mouse system to characterize the in vivo behavior of reservoirs of human immunodeficiency virus type 1-infected cells. J Infect Dis. 186(10):1412-21, 2002.
Wheldon, T.E. Targeting radiation to tumors. Int. J. Radiat. Biol. 65:109-116, 1994.
Xu JY, Gorny MK, Palker T, Karwowska S, Zolla-Pazner S. Epitope mapping of two immunodominant domains of gp41, the transmembrane protein of human immunodeficiency virus type 1, using ten human monoclonal antibodies. J Virol. 65(9): 4832-8, 1991.
Zolla-Pazner, S. et al. Serotyping of primary human immunodeficiency virus type 1 isolates from diverse geographic locations by flow cytometry. J. Virol. 69: 3807-3815, 1995.
Zolla-Pazner S. Identifying epitopes of HIV-1 that induce protective antibodies. Nature Reviews Immunology 4: 199-210, 2004.

## Claims

1. Use of a non-neutralizing radiolabeled monoclonal antibody for the preparation of a composition for killing HIV infected cells, wherein the antibody is specific for HIV gp41 or gp120 envelope glycoprotein, wherein the radiolabel is an alpha emitter or a beta emitter, and wherein the radiolabeled antibody specifically binds to cells that are infected with HIV virus and that express the HIV envelope glycoprotein to which the antibody specifically binds.

2. The use of claim 1, wherein the monoclonal antibody is labeled with an alpha emitter.

3. The use of claim 2, wherein the alpha emitter is selected from the group consisting of 213-Bismuth, 212-Bismuth, 212-Lead and 211-Astatine.

4. The use of claim 2, wherein the alpha emitter is 213-Bismuth.

5. The use of claim 1, wherein the monoclonal antibody is labeled with a beta emitter.

6. The use of claim 5, wherein the beta emitter is selected from the group consisting of 131-Iodine, 90-Yttrium, 188-Rhenium, 186-Rhenium, 177-Luthetium, 166-Holmium, 64-Copper, 67-Copper, and 153-Samarium.

7. The use of claim 5, wherein the beta emitter is 188-Rhenium.

8. The use of any of claims 1-7, wherein the antibody is an IgG antibody, an IgM antibody, or an IgA antibody, or a fragment thereof, or a domain-deleted antibody, wherein the radiolabeled fragment or domain-deleted antibody specifically binds to cells that are infected with HIV virus and that express the HIV envelope glycoprotein T² to which the fragment or domain-delected antibody specifically binds. T² gp41 at gp 120.

9. The use of any of claims 1-7, wherein the antibody is an IgG antibody.

10. The use of any of claims 1-9, wherein the dose of the radioisotope is between 1-500 mCi.

11. The use of any of claims 1-10, wherein the HIV is HIV type 1 or HIV type 2.

12. The use of any of claims 1-10, wherein the HIV is HIV type 1.

13. The use of any of claims 1-12, wherein the HIV-infected cell is a lymphocyte, a T lymphocyte, a monocyte, a macrophage, an astrocyte and/or a microglial cell.

14. The use of any of claims 1-13, wherein the HIV envelope glycoprotein is gp41.

15. The use of any of claims 1-13, wherein the HIV envelope glycoprotein is gp120.

16. A non-neutralizing radiolabeled monoclonal antibody for killing HIV infected cells, wherein the antibody is specific for HIV gp41 or gp120 envelope glycoprotein, wherein the radiolabel is an alpha emitter or a beta emitter, and wherein the radiolabeled antibody specifically binds to cells that are infected with HIV virus and that express the HIV envelope glycoprotein to which the antibody specifically binds.

17. The antibody of claim 16, wherein the monoclonal antibody is labeled with an alpha emitter.

18. The antibody of claim 17, wherein the alpha emitter is selected from the group consisting of 213-Bismuth, 212-Bismuth, 212-Lead and 211-Astatine.

19. The antibody claim 17, wherein the alpha emitter is 213-Bismuth.

20. The antibody of claim 16, wherein the monoclonal antibody is labeled with a beta emitter.

21. The antibody of claim 20, wherein the beta emitter is selected from the group consisting of 131-Iodine, 90-Yttrium, 188-Rhenium, 186-Rhenium, 177-Luthetium, 166-Holmium, 64-Copper, 67-Copper, and 153-Samarium.

22. The antibody of claim 20, wherein the beta emitter is 188-Rhenium.

23. The antibody of any of claims 16-22, wherein the antibody is an IgG antibody, an IgM antibody, or an IgA antibody, or a fragment thereof, or a domain-deleted antibody wherein the radiolabeled fragment or domain-deleted antibody specifically binds to cells that are infected with HIV virus and that express the HIV envelope glycoprotein to which the fragment or domain-deleted antibody specifically brinds. T² gp41 or gp 120,

24. The antibody any of claims 16-22, wherein the antibody is an IgG antibody.

25. The antibody of any of claims 16-24, wherein the dose of the radioisotope is between 1-500 mCi.

26. The antibody any of claims 16-25, wherein the HIV is HIV type 1 or HIV type 2.

27. The antibody any of claims 16-25, wherein the HIV is HIV type 1.

28. The antibody of any of claims 16-27, wherein the HIV-infected cell is a lymphocyte, a T lymphocyte, a monocyte, a macrophage, an astrocyte and/or a microglial cell.

29. The antibody of any of claims 16-28, wherein the HIV envelope glycoprotein is gp41.

30. The antibody of any of claims 16-28, wherein the HIV envelope glycoprotein is gp120.

## Patentansprüche

1. Verwendung eines nicht-neutralisierenden, radioaktiv markierten, monoklonalen Antikörpers zur Herstellung einer Zusammensetzung für die Abtötung von HIV-infizierten Zellen, wobei der Antikörper für das HIV-Hüllglykoprotein gp41 oder das HIV-Hüllglykoprotein gp120 spezifisch ist, wobei der radioaktive Marker ein Alpha-Strahler oder ein Beta-Strahler ist und wobei der radioaktiv markierte Antikörper spezifisch an Zellen bindet, die mit dem HI-Virus infiziert sind und die das HIV-Hüllglykoprotein exprimieren, an das der Antikörper spezifisch bindet.

2. Verwendung nach Anspruch 1, wobei der monoklonale Antikörper mit einem Alpha-Strahler markiert ist.

3. Verwendung nach Anspruch 2, wobei der Alpha-Strahler ausgewählt ist aus der Gruppe, die aus Wismut-213, Wismut-212, Blei-212 und Astatin-211 besteht.

4. Verwendung nach Anspruch 2, wobei der Alpha-Strahler Wismut-213 ist.

5. Verwendung nach Anspruch 1, wobei der monoklonale Antikörper mit einem Beta-Strahler markiert ist.

6. Verwendung nach Anspruch 5, wobei der Beta-Strahler ausgewählt ist aus der Gruppe, die aus Iod-131, Yttrium-90, Rhenium-188, Rhenium-186, Lutetium-177, Holmium-166, Kupfer-64, Kupfer-67 und Samarium-153 besteht.

7. Verwendung nach Anspruch 5, wobei der Beta-Strahler Rhenium-188 ist.

8. Verwendung nach einem der Ansprüche 1 - 7, wobei der Antikörper ein IgG-Antikörper, ein IgM-Antikörper oder ein IgA-Antikörper oder ein Fragment davon ist, oder ein Antikörper mit einer Deletion einer Domäne, wobei das radioaktiv markierte Fragment oder der Antikörper mit einer Deletion einer Domäne spezifisch an Zellen bindet, die mit HI-Virus infiziert sind und die das HIV-Hüllglykoprotein gp41 oder gp120 exprimieren, an die das Fragment oder der Antikörper mit einer Deletion einer Domäne spezifisch bindet.

9. Verwendung nach einem der Ansprüche 1 - 7, wobei der Antikörper ein IgG-Antikörper ist.

10. Verwendung nach einem der Ansprüche 1 - 9, wobei die Dosis von dem Radioisotop zwischen 1 - 500 mCi liegt.

11. Verwendung nach einem der Ansprüche 1 - 10, wobei der HIV entweder vom HIV-Typ 1 oder vom HIV-Typ 2 ist.

12. Verwendung nach einem der Ansprüche 1 - 10, wobei der HIV vom HIV-Typ 1 ist.

13. Verwendung nach einem der Ansprüche 1 - 12, wobei die HIVinfizierte Zelle eine Lymphozyten-, eine T-Lymphozyten-, eine Monozyten-, eine Makrophagen-, eine Astrozyten- und /oder eine Mikroglia-Zelle ist.

14. Verwendung nach einem der Ansprüche 1 - 13, wobei das HIV-Hüllglykoprotein gp41 ist.

15. Verwendung nach einem der Ansprüche 1 - 13, wobei das HIV-Hüllglykoprotein gp120 ist.

16. Nicht-neutralisierender, radioaktiv markierter, monoklonaler Antikörper zur Abtötung von HIV-infizierten Zellen, wobei der Antikörper für das HIV-Hüllglykoprotein gp41 oder das HIV-Hüllglykoprotein gp120 spezifisch ist, wobei der radioaktive Marker ein Alpha-Strahler oder ein Beta-Strahler ist und wobei der radioaktiv markierte Antikörper spezifisch an Zellen bindet, die mit dem HI-Virus infiziert sind und die das HIV-Hüllglykoprotein exprimieren, an das der Antikörper spezifisch bindet.

17. Antikörper nach Anspruch 16, wobei der monoklonale Antikörper mit einem Alpha-Strahler markiert ist.

18. Antikörper nach Anspruch 17, wobei der Alpha-Strahler ausgewählt ist aus der Gruppe, die aus Wismut-213, Wismut-212, Blei-212 und Astatin-211 besteht.

19. Antikörper nach Anspruch 17, wobei der Alpha-Strahler Wismut-213 ist.

20. Antikörper nach Anspruch 16, wobei der monoklonale Antikörper mit einem Beta-Strahler markiert ist.

21. Antikörper nach Anspruch 20, wobei der Beta-Strahler ausgewählt ist aus der Gruppe, die aus Iod-131, Yttrium-90, Rhenium-188, Rhenium-186, Lutetium-177, Holmium-166, Kupfer-64, Kupfer-67 und Samarium-153 besteht.

22. Antikörper nach Anspruch 20, wobei der Beta-Strahler Rhenium-188 ist.

23. Antikörper nach einem der Ansprüche 16 - 22, wobei der Antikörper ein IgG-Antikörper, ein IgM-Antikörper oder ein IgA-Antikörper oder ein Fragment davon ist, oder ein Antikörper mit einer Deletion einer Domäne, wobei das radioaktiv markierte Fragment oder der Antikörper mit einer Deletion einer Domäne spezifisch an Zellen bindet, die mit HI-Virus infiziert sind und die das HIV-Hüllglykoprotein gp41 oder gp120 exprimieren, an die das Fragment oder der Antikörper mit einer Deletion einer Domäne spezifisch bindet.

24. Antikörper nach einem der Ansprüche 16 - 22, wobei der Antikörper ein IgG-Antikörper ist.

25. Antikörper nach einem der Ansprüche 16 - 24, wobei die Dosis von dem Radioisotop zwischen 1 - 500 mCi liegt.

26. Antikörper nach einem der Ansprüche 16 - 25, wobei der HIV entweder vom HIV-Typ 1 oder vom HIV-Typ 2 ist.

27. Antikörper nach einem der Ansprüche 16 - 25, wobei der HIV vom HIV-Typ 1 ist.

28. Antikörper nach einem der Ansprüche 16 - 27, wobei die HIVinfizierte Zelle eine Lymphozyten-, eine T-Lymphozyten-, eine Monozyten-, eine Makrophagen-, eine Astrozyten- und /oder eine Mikroglia-Zelle ist.

29. Antikörper nach einem der Ansprüche 16 - 28, wobei das HIV-Hüllglykoprotein gp41 ist.

30. Antikörper nach einem der Ansprüche 16 - 28, wobei das HIV-Hüllglykoprotein gp120 ist.
* * * * * *

## Revendications

1. Utilisation d'un anticorps monoclonal radiomarqué non neutralisant pour la préparation d'une composition destinée à détruire les cellules infectées par le VIH, l'anticorps étant spécifique de la glycoprotéine d'enveloppe gp41 ou gp120 du VIH, le marqueur radioactif étant un émetteur alpha ou un émetteur bêta, et l'anticorps radiomarqué se liant spécifiquement aux cellules qui sont infectées par le VIH et qui expriment la glycoprotéine d'enveloppe du VIH à laquelle l'anticorps se lie spécifiquement.

2. Utilisation selon la revendication 1, l'anticorps monoclonal étant marqué avec un émetteur alpha.

3. Utilisation selon la revendication 2, l'émetteur alpha étant choisi dans le groupe constitué par le bismuth 213, le bismuth 212, le plomb 212 et l'astatine 211.

4. Utilisation selon la revendication 2, l'émetteur alpha étant le bismuth 213.

5. Utilisation selon la revendication 1, l'anticorps monoclonal étant marqué avec un émetteur bêta.

6. Utilisation selon la revendication 5, l'émetteur alpha étant choisi dans le groupe constitué par l'iode 131, l'yttrium 90, le rhénium 188, le rhénium 186, le lutétium 177, l'holmium 166, le cuivre 64, le cuivre 67, et le samarium 153.

7. Utilisation selon la revendication 5, l'émetteur bêta étant le rhénium 188.

8. Utilisation selon l'une quelconque des revendications 1 à 7, l'anticorps étant un anticorps IgG, un anticorps IgM, ou un anticorps IgA, ou un fragment de ceux-ci, ou un anticorps à domaine délété, le fragment ou l'anticorps à domaine délété radiomarqués se liant spécifiquement aux cellules qui sont infectées par le VIH et qui expriment la glycoprotéine d'enveloppe du VIH gp41 ou gp120 à laquelle le fragment ou l'anticorps à domaine délété se lient spécifiquement.

9. Utilisation selon l'une quelconque des revendications 1 à 7, l'anticorps étant un anticorps IgG.

10. Utilisation selon l'une quelconque des revendications 1 à 9, la dose du radioisotope étant comprise entre 1 et 500 mCi.

11. Utilisation selon l'une quelconque des revendications 1 à 10, le VIH étant le VIH de type 1 ou le VIH de type 2.

12. Utilisation selon l'une quelconque des revendications 1 à 10, le VIH étant le VIH de type 1.

13. Utilisation selon l'une quelconque des revendications 1 à 12, la cellule infectée par le VIH étant un lymphocyte, un lymphocyte T, un monocyte, un macrophage, un astrocyte et/ou une cellule microgliale.

14. Utilisation selon l'une quelconque des revendications 1 à 13, la glycoprotéine d'enveloppe du VIH étant la gp41.

15. Utilisation selon l'une quelconque des revendications 1 à 13, la glycoprotéine d'enveloppe du VIH étant la gp120.

16. Anticorps monoclonal radiomarqué non neutralisant destiné à détruire les cellules infectées par le VIH, l'anticorps étant spécifique de la glycoprotéine d'enveloppe gp41 ou gp120 du VIH, le marqueur radioactif étant un émetteur alpha ou un émetteur bêta, et l'anticorps radiomarqué se liant spécifiquement aux cellules qui sont infectées par le VIH et qui expriment la glycoprotéine d'enveloppe du VIH à laquelle l'anticorps se lie spécifiquement.

17. Anticorps selon la revendication 16, l'anticorps monoclonal étant marqué avec un émetteur alpha.

18. Anticorps selon la revendication 17, l'émetteur alpha étant choisi dans le groupe constitué par le bismuth 213, le bismuth 212, le plomb 212 et l'astatine 211.

19. Anticorps selon la revendication 17, l'émetteur alpha étant le bismuth 213.

20. Anticorps selon la revendication 16, l'anticorps monoclonal étant marqué avec un émetteur bêta.

21. Anticorps selon la revendication 20, l'émetteur alpha étant choisi dans le groupe constitué par l'iode 131, l'yttrium 90, le rhénium 188, le rhénium 186, le lutétium 177, l'holmium 166, le cuivre 64, le cuivre 67, et le samarium 153.

22. Anticorps selon la revendication 20, l'émetteur bêta étant le rhénium 188.

23. Anticorps selon l'une quelconque des revendications 16 à 22, l'anticorps étant un anticorps IgG, un anticorps IgM, ou un anticorps IgA, ou un fragment de ceux-ci, ou un anticorps à domaine délété, le fragment ou l'anticorps à domaine délété radiomarqués se liant spécifiquement aux cellules qui sont infectées par le VIH et qui expriment la glycoprotéine d'enveloppe du VIH gp41 ou gp120 à laquelle le fragment ou l'anticorps à domaine délété se lient spécifiquement.

24. Anticorps selon l'une quelconque des revendications 16 à 22, l'anticorps étant un anticorps IgG.

25. Anticorps selon l'une quelconque des revendications 16 à 24, la dose du radioisotope étant comprise entre 1 et 500 mCi.

26. Anticorps selon l'une quelconque des revendications 16 à 25, le VIH étant le VIH de type 1 ou le VIH de type 2.

27. Anticorps selon l'une quelconque des revendications 16 à 25, le VIH étant le VIH de type 1.

28. Anticorps selon l'une quelconque des revendications 16 à 27, la cellule infectée par le VIH étant un lymphocyte, un lymphocyte T, un monocyte, un macrophage, un astrocyte et/ou une cellule microgliale.

29. Anticorps selon l'une quelconque des revendications 16 à 28, la glycoprotéine d'enveloppe du VIH étant la gp41.

30. Anticorps selon l'une quelconque des revendications 16 à 28, la glycoprotéine d'enveloppe du VIH étant la gp120.
